# EUROPEAN PATENT SPECIFICATION

(11) **EP 0 609 455 B1**
(45) Date of publication and mention of the grant of the patent: **11.03.1998**
(21) Application number: 93914935.7
(22) Date of filing: 30.06.1993
(51) Int. Cl.: C07C 37/08, C07C 37/86, C07C 39/07, C07C 409/08

(54) **PROCESS FOR PRODUCING CRESOLS**
VERFAHREN ZUR HERSTELLUNG VON KRESOLEN
PROCEDE DE PRODUCTION DE CRESOLS

(30) Priority: 01.07.1992 JP 174222/92
(43) Date of publication of application: 10.08.1994
(73) Proprietor: SUMITOMO CHEMICAL COMPANY LIMITED, Osaka-shi, Osaka 541 (JP)
(72) Inventor: IKIMI, Kiyoshi, Oita-shi, Oita 870 (JP); IKEDA, Yoichi, Oita-shi, Oita 870-01 (JP); MURAKAMI, Akira, Oita-shi, Oita 870-02 (JP); OKAMOTO, Kazushige, Oita-shi, Oita 870-01 (JP); TOKUMARU, Tooru, Oita-shi, Oita 870-01 (JP); HAZAMA, Motoo, Oita-shi, Oita 870 (JP)
(74) Representative: VOSSIUS & PARTNER
(86) International application number: JP9300898
(87) International publication number: WO9401387

(56) References cited:
- DE-A- 1 803 036
- DE-A- 2 650 416
- JP-A- 4 880 522
- JP-A- 5 879 941
- JP-A- 6 054 357
- JP-A- 6 335 558
- JP-A-50 130 726
- CHEMICAL ABSTRACTS, vol. 88, no. 25, 19 June 1978, Columbus, Ohio, US; abstract no. 190380, page 722 ;column 1 ; & JP-A-78 012 825 (MITSUI PETROCHEMICAL INDUSTRIES)

## Description

The present invention relates to a process for the production of cresols.

As a process for cresol production, there has been widely known a process in which cymene is oxygenated with oxygen and the resulting tertiary hydroperoxide is decomposed into cresols and acetone.

In this process, however, primary hydroperoxide of cymene with an oxygenated methyl group is formed in the oxygenation, together with the above tertiary hydroperoxide. The primary hydroperoxide gives isopropylphenol and formaldehyde by its decomposition. This formaldehyde may be condensed with the resulting cresols to form a resin, which will cause a decrease in the yield of cresols in this process.

To solve this problem, a process has been proposed, in which the decomposition is stopped halfway after the oxygenation, followed by hydrogenation (JP-A 52-57130, JP-B 59-8246, JP-B 1-49248).

This process is, however, not satisfactory with respect to the yield of cresols formed.

Further processes for the production of cresols are disclosed in EP-A-077749 and Chemical Abstracts, vol. 88, no. 25 (19.6.1978), abstract no. 190380m.

For the purpose of preventing the formation of formaldehyde as described above, there has been proposed a process in which a mixture of the tertiary hydroperoxide and primary hydroperoxide obtained by the oxygenation is reacted with an alkali and an organic quaternary ammonium salt to reduce the content of primary hydroperxide (JP-A 63-35558). It cannot always, however, be said that this process reaches the satisfactory level from the viewpoint of an improvement in the yield of cresols.

Thus, the present inventors have intensively studied a production process for cresols. As the result, they have found that by reacting a solution of oxygenation products containing tertiary and primary hydroperoxides obtained by oxygenation of cymene with oxygen, in advance of decomposition, with an organic quaternary ammonium salt and an alkali, or with an organic quaternary ammonium hydroxide to reduce the content of primary hydroperoxide, followed by decomposition, after which the decomposition mixture is subjected to hydrogenation, it is possible not only to obtain cresols in high yield but also to attain selective recovery of primary hydroperoxide and other by-products as the raw material cymene, thereby completing the present invention.

The present invention provides a process for the production of cresols, characterized in that the cresols are obtained by the steps of:
(1) conducting oxygenation of cymene with oxygen to obtain a solution of oxygenation products containing tertiary hydroperoxide and primary hydroperoxide thereof;
(2) reacting the solution of oxygenation products with an organic quaternary ammonium salt and an alkali, or with an organic quaternary ammonium hydroxide to reduce the content of primary hydroperoxide;
(3) subjecting the reaction mixture to decomposition in the presence of a catalyst; and
(4) subjecting the decomposition mixture to hydrogenation.

The present invention will hereinafter be explained in detail.

First, the following will describe the step of conducting oxygenation of cymene with oxygen to obtain a solution of oxygenation products containing tertiary hydroperoxide and primary hydroperoxide thereof.

As the cymene to be used in this step, there can be mentioned o-cymene, m-cymene and p-cymene. These compounds can be used alone or in admixture with each other at any proportion.

This step can be performed by ordinary oxygenation in liquid phase, and usually attained by bringing cymene in contact with oxygen gas or an oxygen-containing gas.

This step is usually performed under normal pressure or under pressure. The pressure is usually in the range of from 0 to 1,96 MPa (0 to 20 kg/cm²) as a gauge pressure.

In this step, any of alkalis or their aqueous solutions may be allowed to coexist, examples of which are carbonates of alkali metals, such as sodium carbonate and potassium carbonate; hydroxides and carbonates of alkaline earth metals, such as magnesium hydroxide, calcium hydroxide and calcium carbonate; amines such as pyridine, piperidine and triethylamine; and ammonia.

In this step, as a reaction initiator, for example, an azo compound such as 2,2'-azobisisobutyronitrile, or a peroxide such as benzoyl peroxide or cymene hydroperoxide may be added. The amount thereof is usually in the range of from 0.01 to 5 wt%, to cymene.

The reaction temperature is usually in the range of from 30° to 200°C, preferably 80° to 150°C.

The ratio of formed primary hydroperoxide to formed tertiary hydroperoxide is usually in the range of from 5-30/95-70 (primary/tertiary; the sum total of both is 100).

After the reaction, a solution of oxygenation products is obtained, which may be subjected, if necessary, to a treatment such as fractionation or filtration. This solution of oxygenation products can be used in the subsequent step without undergoing a particular post-treatment; in case where no alkali is used in this step, however, the resulting solution of oxygenation products may be washed, before its use in the subsequent step, with an aqueous solution of alkali metal hydroxides, alkali metal carbonates, alkaline earth metal hydroxides or alkaline earth metal carbonates.

The following will describe the step of reacting the solution of oxygenation products obtained above with an organic quaternary ammonium salt and an alkali, or with an organic quaternary ammonium hydroxide.

As the organic quaternary ammonium salt to be used in this reaction, there can be exemplified a compound of the general formula [1]: 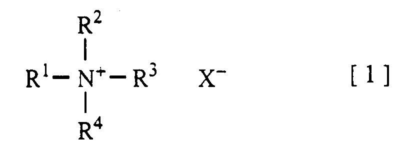 (wherein R¹ and R² are independently an alkyl group optionally having any substituent or an aralkyl group optionally having any substituent; R³ and R⁴ are independently an alkyl group; and X is an anionic residue); and a compound of the general formula [2]: 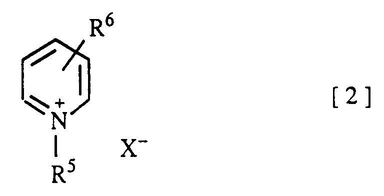 (wherein R⁵ is an alkyl group optionally having any substituent; R⁶ is a hydrogen atom or an alkyl group; and X is as defined above).

In the compounds as described above, as the anionic residue, there can be mentioned, for example, halogen atoms such as chlorine, bromine and iodine; H₂PO₄, CH₃COO, CH₃OSO₃, C₂H₅OSO₃, ClO₄ and HSO₄ groups.

As the compound [1] or the compound [2], there can be mentioned, for example, tetramethylammonium chloride, tetraethylammonium chloride, tetra-n-propylammonium chloride, tetra-n-butylammonium chloride, benzyltrimethylammonium chloride, benzyltriethylammonium chloride, stearyltrimethylammonium chloride, trimethyloctadecylammonium chloride, lauryltrimethylammonium chloride, trimethylhexadecylammonium chloride, distearyldimethylammonium chloride, dicetyldimethylammonium chloride, tricaprylmethylammonium chloride, o-, m- or p-methoxybenzyltriethylammonium chloride, o-, m- or p-phenoxybenzyltriethylammonium chloride, trimethyldodecylammonium chloride, trimethyldecylammonium chloride, trioctylmethylammonium chloride, N-butylpyridinium chloride, N-laurylpyridinium chloride, N-laurylpicolinium chloride, triethylpropylammonium chloride, diethylpropylbenzylammonium chloride, o-, m-or p-chlorobenzylthethylarrmonium chloride, methylethylpropylbenzylammonium chloride, diethylbutylbenzylammonium chloride, methyldiethylbenzylammonium chloride, dimethylethylbenzylammonium chloride, tripropylbenzylammonium chloride, ethyldipropylbenzylammonium chloride, diethyldibenzylammonium chloride, dimethyllaurylbenzylammonium chloride, dimethylstearylbenzylammonium chloride, dimethyloctylbenzylammonium chloride, dimethylmyristylbenzylammonium chloride, as well as bromides, iodides, perchlorates, dihydrogenphosphates, hydrogensulfates, methylsulfates and ethylsulfates corresponding to these chlorides. These compounds can be used alone or in admixture with each other. The amount thereof is usually in the range of from 0.001 to 1 time in mole, preferably from 0.001 to 0.5 times in mole, to the primary hydroperoxide.

As the alkali to be used together with the organic quaternary ammonium salt, there can be mentioned, for example, alkali metal hydroxides and alkaline earth metal hydroxides, such as sodium hydroxide, potassium hydroxide, lithium hydroxide, calcium hydroxide, barium hydroxide, magnesium hydroxide and strontium hydroxide. The amount thereof is usually in the range of from 0.1 to 20 times in mole, preferably from 0.5 to 10 times in mole, to the primary hydroperoxide.

In case where an organic quaternary ammonium hydroxide is used in the reaction, as the organic quaternary ammonium hydroxide, there can be exemplified, for example, a compound of the general formula [3]: 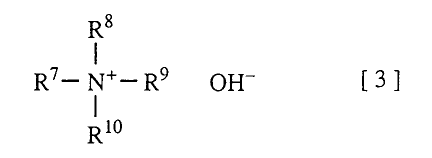 (wherein R⁷ and R⁸ are independently an alkyl group optionally having any substituent or an aralkyl group optionally having any substituent; and R⁹ and R¹⁰ are independently an alkyl group); and a compound of the general formula [4]: 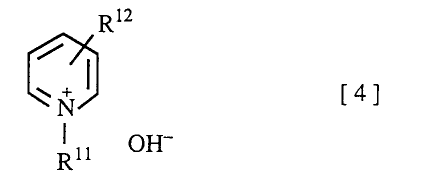 (wherein R¹¹ is an alkyl group optionally having any substituent; R¹² is a hydrogen atom or an alkyl group).

As the compound [3] or the compound [4], there can be mentioned, for example, tetramethylammonium hydroxide, tetraethylammonium hydroxide, tetra-n-propylammonium hydroxide, tetra-n-butylammonium hydroxide, benzyltrimethylammonium hydroxide, benzyltriethylammonium hydroxide, stearyltrimethylammonium hydroxide, trimethyloctadecylammonium hydroxide, lauryltrimethylammonium hydroxide, trimethylhexadecylammonium hydroxide, distearyldimethylammonium hydroxide, dicetyldimethylammonium hydroxide, tricaprylmethylammonium hydroxide, o-, m- or p-methoxybenzyltriethylammonium hydroxide, o-, m- or p-phenoxybenzyltriethylammonium hydroxide, trimethyldodecylammonium hydroxide, trimethyldecylammonium hydroxide, trioctylmethylammonium hydroxide, N-butylpyridinium hydroxide, N-laurylpyridinium hydroxide, N-laurylpicolinium hydroxide, triethylpropylammonium hydroxide, diethylpropylbenzylammonium hydroxide, o-, m- or p-chlorobenzyltriethylammonium hydroxide, methylethylpropylbenzylammonium hydroxide, diethylbutylbenzylammonium hydroxide, methyldiethylbenzylammonium hydroxide, dimethylethylbenzylammonium hydroxide, tripropylbenzylammonium hydroxide, ethyldipropylbenzylammonium hydroxide, diethyldibenzylammonium hydroxide, dimethyllaurylbenzylammonium hydroxide, dimethylstearylbenzylammonium hydroxide, dimethyloctylbenzylammonium hydroxide and dimethylmyristylbenzylammonium hydroxide. These compounds can be used alone or in admixture with each other.

The amount thereof is usually in the range of from 0.001 to 5.0 times in mole to the primary hydroperoxide.

In case where an organic quaternary ammonium hydroxide is used, it is preferred to combine an alkali therewith, because the amount of organic quaternary ammonium hydroxide to be used can be decreased. The amount of organic quaternary ammonium hydroxide to be used together with an alkali is usually in the range of from 0.001 to 1 time in mole, preferably 0.001 to 0.5 times in mole, to the primary hydroperoxide. The amount of organic quaternary ammonium hydroxide to be used with no alkali is usually in the range of from 0.1 to 5 times in mole, preferably 0.3 to 3 times in mole, to the primary hydroperoxide. As the alkali in case where the alkali is used together, there can be mentioned, for example, alkali metal hydroxides and alkaline earth metal hydroxides, such as sodium hydroxide, potassium hydroxide, lithium hydroxide, calcium hydroxide, barium hydroxide, magnesium hydroxide and strontium hydroxide. The amount thereof is usually in the range of from 0.1 to 20 times in mole, preferably from 0.5 to 10 times in mole, to the primary hydroperoxide.

This step is usually conducted in the presence of a polar solvent such as water, methanol or ethanol. Preferably used is water.

The reaction temperature is usually in the range of from 30° to 150°C, preferably from 70° to 110°C.

In this step, it is favorable to effect the reaction so that the content of primary hydroperoxide to tertiary hydroperoxide is usually decreased to not greater than 1/25 (w/w), preferably not greater than 1/50 (w/w), and more preferably not greater than 1/100 (w/w), at which time it is more favorable to suppress the degree of conversion of tertiary hydroperoxide so as to become 20 % or less, preferably 10 % or less, and more preferably 5 % or less.

In this step, the end point of the reaction can be determined, for example, from the analysis of the reaction mixture by liquid chromatography to check the content ratio of primary hydroperoxide to tertiary hydroperoxide.

After the reaction, for example, the reaction mixture is allowed to stand and subjected to fractionation, washing with water, before the subsequent step. Thus, it is possible not only to suppress the formation of isopropylphenol and formaldehyde from the primary hydroperoxide in the subsequent step but also to convert the primary hydroperoxide and other by-products into the compounds capable of being recovered as cymene.

The following will describe the step of subjecting the reaction mixture obtained above to decomposition in the presence of a catalyst.

As the catalyst to be used in this step, there can be mentioned, for example, acidic catalysts, sulfur and Burmah catalysts. As the acidic catalyst, there can be mentioned, for example, inorganic acids such as sulfuric acid, hydrochloric acid, perchloric acid, SO₂ and SO₃; organic acids such as benzenesulfonic acid, p-toluenesulfonic acid, cresolsulfonic acid and chloroacetic acid; solid acids such as silica-alumina, alumina and acidic ion exchange resins; heteropolyacids such as tungstosilicic acid, tungstophosphoric acid and molybdophosphoric acid. The Burmah catalyst refers to a catalyst carrying a metal complex of the general formula [5]: 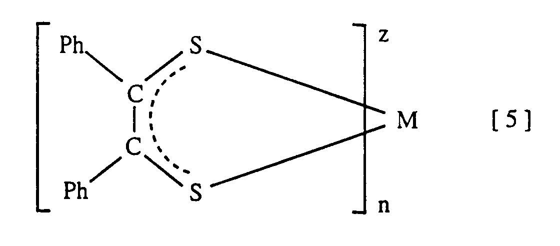 (wherein M is Ni, Pd or Fe (II); Ph is a phenyl group optionally having any substituent; n is an integer of 1 ,2 or 3; z is a formal charge of the complex, 0, -1 or -2), and there can be mentioned, for example, bis(dithiobenzil)nickel, bis(dithiobenzil)palladium and bis(dithiobenzil)iron (II). Preferred as the catalyst are sulfuric acid and cresolsulfonic acid.

The amount of catalyst to be used, although it can be suitably determined depending upon the kind of that catalyst, is usually in the range of from about 0.0001 to 1 wt%, to the reaction mixture to be treated.

The reaction temperature is usually in the range of from 30° to 150°C.

In this step, the mixture thus treated can be subjected to an analysis such as liquid chromatography to check the degree of decomposition of hydroperoxides.

After the reaction, the reaction mixture can be subjected to the subsequent step without undergoing a particular post-treatment, or if necessary, after subjected to an operation such as filtration and neutralization.

Alternatively, the reaction mixture obtained may also be subjected to the subsequent step after the removal of produced acetone therefrom.

The following will describe the step of subjecting the decomposition mixture obtained above to hydrogenation. This step can be performed by ordinary catalytic hydrogenation, and usually attained by introducing hydrogen gas under normal pressure or under pressure in the presence of a catalyst. The pressure is usually in the range of from 0 to 9,8 MPa (0 to 100 kg/cm²) as a gauge pressure.

As the catalyst to be used, there can be mentioned, for example, catalysts such as Pd, Cr, Cu, Pt, Ni, Ru, Rh and Re. These catalysts can also be used in a supported form on a carrier such as active carbon, titania, zirconia, silica-magnesia, alumina, alumina-magnesia or acidic ion exchange resin. Preferably, a Pd catalyst or a copper-chromium catalyst is used, and more preferably, Pd/C, Pd/alumina, Pd/TiO₂, copper-chromium/C, copper-chromium/TiO₂ or Pd/acidic ion exchange resin is used. The amount of catalyst to be used is usually in the range of from 0.001 to 20 wt% to the decomposition mixture to be treated.

In this step, any acidic catalyst can also be allowed to coexist, if necessary. As the acidic catalyst, the same catalysts as those used in the step of decomposition as described above can be used. The decomposition mixture may be used in this step as it is, without undergoing the removal of the catalyst used in the decomposition step.

The reaction temperature is usually in the range of from 0° to 250°C, preferably from 20° to 250°C.

After complement of the reaction, cresols, cymene and acetone can be obtained by filtration of the catalyst, and cresols, cymene and acetone can be separated and purified by neutralization, if necessary, and then distillation. The cymene obtained can be recycled for use as the raw material of the present invention.

The present invention can be performed either by a batch or a continuous method.

According to the process of the present invention, it is possible to obtain cresols in high yield by conducting oxygenation treatment of cymene with oxygen, followed by treatment in the presence of an organic quaternary ammonium salt and an alkali, or in the presence of an organic quaternary ammonium hydroxide, to reduce the content of primary hydroperoxide, decomposition and then hydrogenation,. Moreover, it becomes easy to separate the cresols from the by-products.

The present invention will be further illustrated by way of the following examples which are not to be construed to limit the scope thereof.

The structures represented by the respective abbreviated names for the compounds used in Examples are as follows:

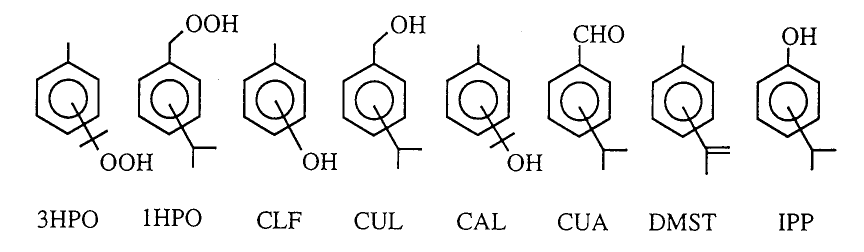

### Example 1

(1) In a reaction vessel equipped with a stirrer,an air-blowing tube, a thermometer and a condenser, placed are 5045 parts by weight of cymene (composition - cymene: 98.3%, 3HPO: 1.10%, 1HPO: 0.032%, CAL: 0.122%, CUL: 0.007%, CUA: 0.0540%, CLF: 0.007%) containing 1.13% cymene hydroperoxide as a reaction initiator. The reaction is conducted, while blowing air thereinto, with stirring at 120°C under normal pressure for 6 hours. After the reaction, 5116 parts by weight of an oxygenation solution (composition: cymene: 86.2%, 3HPO: 9.28%, 1HPO: 1.68%, CAL: 0.620%, CUL: 0.073%, CUA: 0.125%, CLF: 0.006%) are obtained.
(2) Then, 500 parts by weight of the oxygenation solution obtained in (1) are placed in a reaction vessel, to which 0.507 parts by weight of benzyltriethylammonium chloride and 49.5 parts by weight of 8% aqueous sodium hydroxide are added, and the mixture is stirred at 80°C under an atmosphere of nitrogen for 2.5 hours. The mixture is allowed to stand and the water phase is removed therefrom by fractionation to give 498 parts by weight of the organic phase (composition: cymene: 86.3%, 3HPO: 9.22%, 1HPO: 0.005%, CAL: 0.953%, CUL: 0.114%, CUA: 1.69%, CLF: 0.001%). The ratio of 1HPO content to 3HPO content in the organic phase is 1/1844, and the degree of conversion is 99.7% for 1HPO and 0.972% for 3HPO.
   To a reaction vessel charged with 488 parts by weight of the resulting organic phase, added are 50.0 parts by weight of water, and the mixture is stirred for washing at 80°C for 1 hour. Then, the mixture is allowed to stand and the water phase is removed therefrom by fractionation to give 487 parts by weight of the washed organic phase (composition: cymene: 86.4%, 3HPO: 9.36%, 1HPO: 0.007%, CAL: 0.964%, CUL: 0.130%, CUA: 1.67%, CLF: 0.001%).
   Then, 477 parts by weight of the resulting washed organic phase are concentrated at 70°C under a reduced pressure of 10 to 20 mmHg to recover 391 parts by weight of cymene (cymene content, 98.7%) as a distillate and to give 83.9 parts by weight of condensed oil (composition: cymene: 30.5%, 3HPO: 51.9%, 1HPO: 0.0710%, CAL: 5.46%, CUL: 0.620%, CUA: 9.17%, CLF: not found) as a bottom residue.
(3) Then, 20.0 parts by weight of the concentrated oil obtained in (2) are added to a mixture of 0.00803 parts by weight of sulfuric acid and 2.01 parts by weight of acetone under reflux with stirring. After completion of the addition, the mixture is maintained at 65°C for 15 minutes to give 21.7 parts by weight of the decomposition mixture (composition: cymene: 28.4%, 3HPO: not found, 1HPO: not found, CAL: 0.417%, CUL: 1.57%, CUA: 7.31%, CLF: 30.0%, DMST: 3.00%, others: 29.3%). The degree of conversion of 3HPO is 100% and the yield of CLF is 96.6% from 3HPO before the acid decomposition. The total yield of cresol from consumed cymene over a process from the oxygenation step to this step is 58.8%.
(4) In an autoclave made of stainless steel, placed are 15.0 parts by weight of the decomposition mixture obtained in (3), 0.300 parts by weight of 5% palladium-titania catalyst and 0.294 parts by weight of an acidic ion exchange resin (Rohm & Haas Co. - trade name, Amberlyst 15). The hydrogenation is conducted, while introducing hydrogen gas thereinto under a hydrogen gauge pressure of 0,49 MPa (5 kg/cm²), at 40°C for 2 hours and then at 75°C for 1 hour. After the reaction, the catalyst is removed by filtration, and the reaction mixture is neutralized by addition of aqueous sodium hydroxide until the water phase has pH 7. The reaction mixture is allowed to stand and the water phase is removed therefrom by fractionation to give 15.0 parts by weight of the reaction mixture (composition: cymene: 42.8%, 3HPO: not found, 1HPO: not found, CAL: 0.0730%, CUL: 0.161%, CUA: not found, CLF: 30.7%, DMST: 0.363%, others: 25.9%). The total yield of cresol is 78.2% (from the amount of consumed cymene).
   The degree of conversion of (CAL + DMST + CUA + CUL) in this reduction step is 95.0%, the yield of cymene is 126.1% [from the amount of (CAL + DMST + CUA + CUL) before the hydrogenation], and the recovery of cresol is 102.3% (from cresol before the hydrogenation).

### Examples 2-10

Using the oxygenation mixture obtained in (1) of Example 1, the treatment of a hydroperoxide mixture with an organic quaternary ammonium salt or with an organic quaternary ammonium hydroxide is conducted in the same manner as described in (2) of Example 1, except for the conditions shown in Table 1. The results are shown in Table 1.

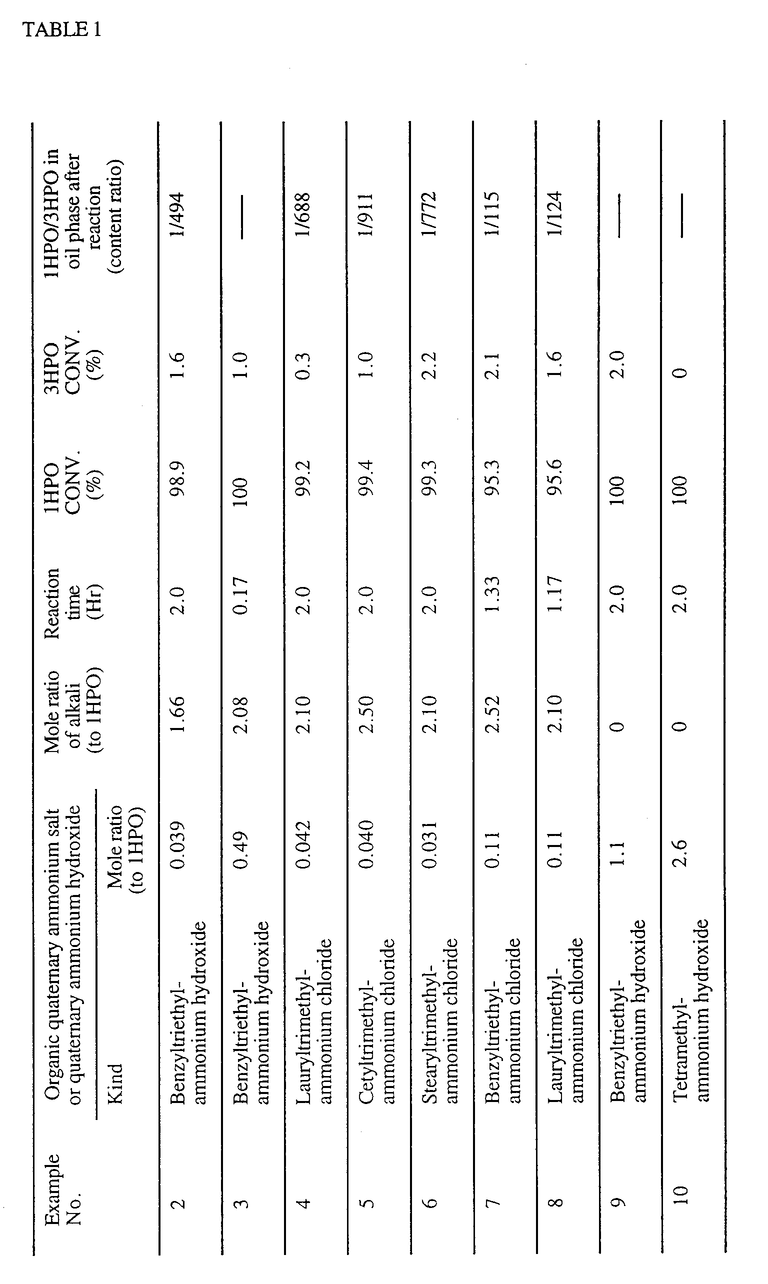

The treated mixture obtained in the foregoing step is washed with water and concentrated, after which it is subjected to the same operations as described in (3) to (4) of Example 1, thereby obtaining cresols and cymene in high yield.

### Example 11

First, 250 parts by weight of the oxygenated oil (composition: cymene: 85.2%, 3HPO: 9.84%, 1HPO: 1.58%, CAL: 0.873%, CUL: 0.142%, CUA: 0.153%, CLF: 0.015%) obtained by the method in (1) of Example 1 are stirred together with 25.0 parts by weight of 4% aqueous sodium hydroxide under an atmosphere of nitrogen at the reaction temperature of 70°C for 0.25 hours to give 250 parts by weight of neutralized oil (composition: cymene: 85.2%, 3HPO: 9.77%, 1HPO: 1.39%, CAL: 0.909%, CUL: 0.220%, CUA: 0.233%, CLF: 0.003%).

Using 200 parts by weight of this neutralized oil, to which 19.9 parts by weight of 8% aqueous sodium hydroxide and 0.599 parts by weight of benzyltriethylammonium chloride are added, the mixture is stirred under an atmosphere of nitrogen at the reaction temperature of 70°C for 0.25 hours. The reaction mixture is allowed to stand and the water phase is removed therefrom by fractionation to give 200 parts by weight of organic phase (composition: cymene: 85.2%, 3HPO: 9.57%, 1HPO: 0.052%, CAL: 1.06%, CUL: 0.317%, CUA: 1.76%, CLF: 0.003%).

The ratio of 1HPO content to 3HPO content in the organic phase is 1/185, and the degree of conversion is 96.3% for 1HPO and 2.03% for 3HPO.

### Examples 12-21

Using the decomposition mixture obtained in (1) to (3) of Example 1, the hydrogenation is conducted in the same manner as described in (4) of Example 1, except for the conditions shown in Table 2. The results are shown in Table 2.

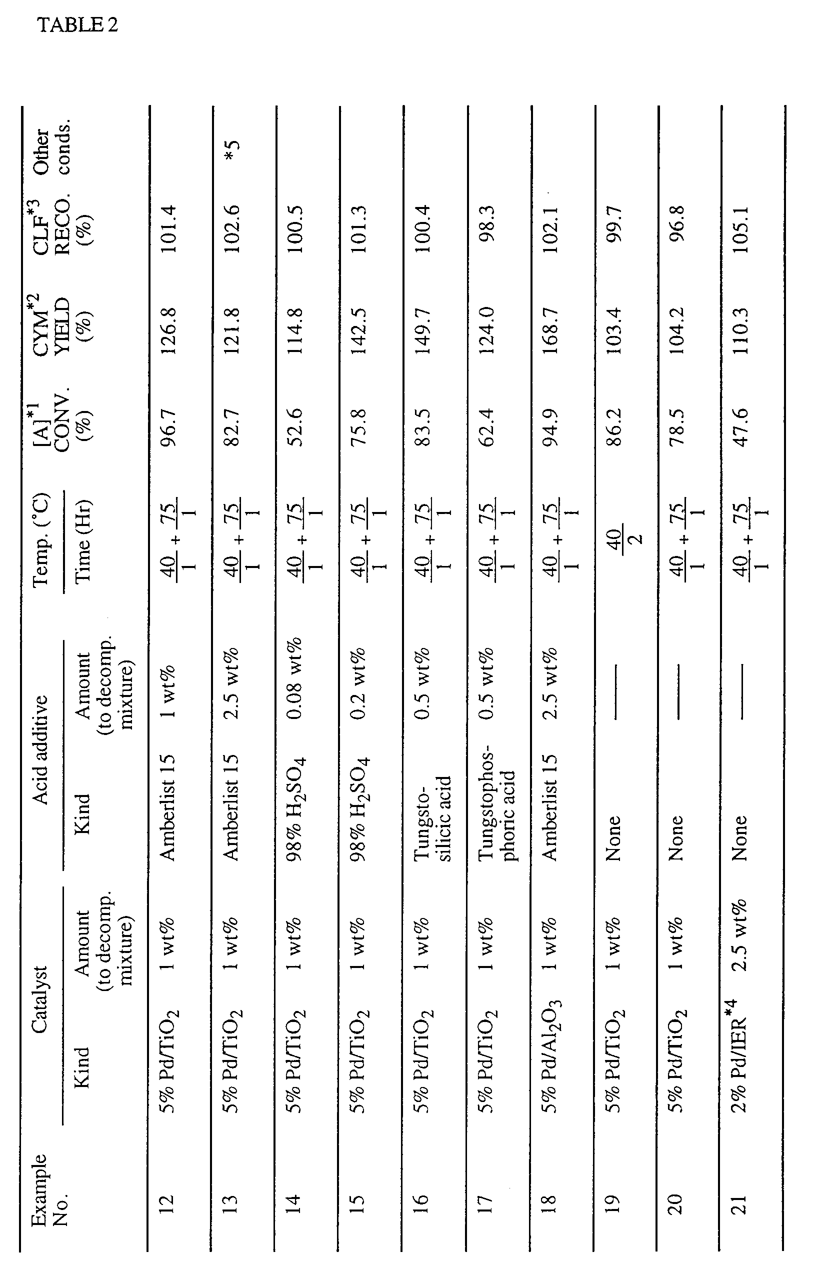

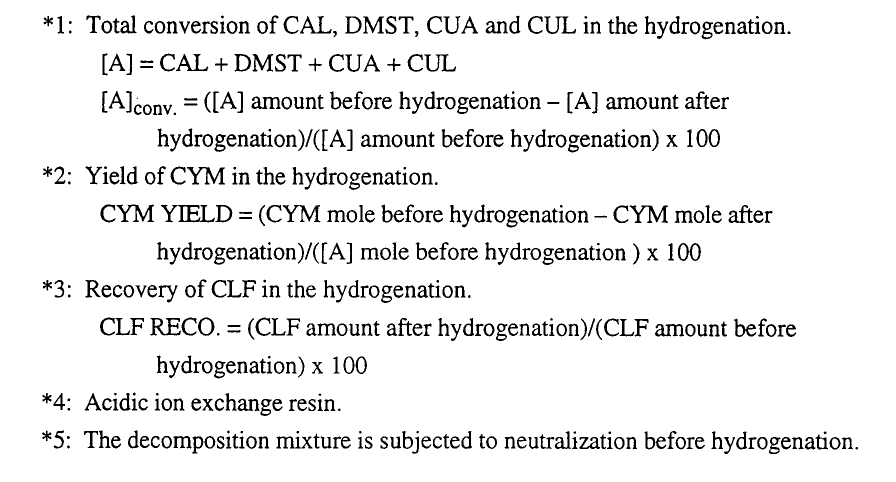

## Claims

1. A process for the production of cresols, characterized in that the cresols are obtained by the steps of:
(1) conducting oxygenation of cymene with oxygen to give a solution of oxygenation products containing tertiary hydroperoxide and primary hydroperoxide thereof;
(2) reacting the solution of oxygenation products with an organic quaternary ammonium salt and an alkali, or with an organic quaternary ammonium hydroxide to reduce the content of primary hydroperoxide;
(3) subjecting the reaction mixture to decomposition in the presence of a catalyst; and
(4) subjecting the decomposition mixture to hydrogenation.

2. A process according to claim 1, wherein the reaction in the step (2) is conducted so that the amount of remaining primary hydroperoxide to tertiary hydroperoxide is decreased to not greater than 1/25 (w/w).

3. A process according to claim 2, wherein the degree of conversion of tertiary hydroperoxide in the step (2) is 20% or less.

4. A process according to claim 1, 2 or 3, wherein an organic quaternary ammonium salt and an alkali are used in the step (2).

5. A process according to claim 1, 2, or 3, wherein an organic quaternary ammonium hydroxide is used together with an alkali in the step (2).

6. A process according to claim 1, 2, 3, 4 or 5, wherein the hydrogenation catalyst is a Pd catalyst or a copper-chromium catalyst in the step (4).

## Patentansprüche

1. Verfahren zur Herstellung von Cresolen, dadurch gekennzeichnet, daß die Cresole durch folgende Schritte erhalten werden:
(1) Durchführen einer Oxidation von Cymol mit Sauerstoff, wobei eine Lösung von Oxidationsprodukten erhalten wird, die das tertiäre Hydroperoxid und primäre Hydroperoxid davon enthält;
(2) Umsetzung der Lösung der Oxidationsprodukte mit einem organischen quaternären Ammoniumsalz und einer Base oder mit einem organischen quaternären Ammoniumhydroxid, um den Gehalt des primären Hydroperoxids zu vermindern;
(3) Zersetzung des Reaktionsgemisches in Gegenwart eines Katalysators; und
(4) Hydrierung des Zersetzungsgemisches.

2. Verfahren nach Anspruch 1, wobei die Umsetzung im Schritt (2) so durchgeführt wird, daß die Menge des verbleibenden primären Hydroperoxids zum tertiären Hydroperoxid auf nicht mehr als 1/25 (Gew./Gew.) vermindert wird.

3. Verfahren nach Anspruch 2, wobei der Grad der Umwandlung des tertiären Hydroperoxids im Schritt (2) 20 % oder weniger beträgt.

4. Verfahren nach Anspruch 1, 2 oder 3, wobei ein organisches quaternäres Ammoniumsalz und eine Base im Schritt (2) verwendet werden.

5. Verfahren nach Anspruch 1, 2 oder 3, wobei ein organisches quaternäres Ammoniumhydroxid zusammen mit einer Base im Schritt (2) verwendet wird.

6. Verfahren nach Anspruch 1, 2, 3, 4 oder 5, wobei der Hydrierungskatalysator im Schritt (4) ein Pd-Katalysator oder ein Kupfer-Chromkatalysator ist.

## Revendications

1. Procédé de production de crésols, caractérisé en ce que les crésols sont obtenus par les étapes consistant :
(1) à effectuer une oxygénation de cymène avec de l'oxygène pour donner une solution de produits d'oxygénation contenant de l'hydroperoxyde tertiaire et de l'hydroperoxyde primaire de cymène;
(2) à faire réagir la solution de produits d'oxygénation avec un sel d'ammonium quaternaire organique et une base, ou avec un hydroxyde d'ammonium quaternaire organique pour réduire la teneur en hydroperoxyde primaire ;
(3) à soumettre le mélange réactionnel à une décomposition en présence d'un catalyseur ; et
(4) à soumettre le mélange de la décomposition à une hydrogénation.

2. Procédé selon la revendication 1, dans lequel la réaction dans l'étape (2) est effectuée de sorte que le rapport hydroperoxyde primaire restant à hydroperoxyde tertiaire est réduit à pas plus de 1/25 (p/p).

3. Procédé selon la revendication 2, dans lequel le degré de conversion de l'hydroperoxyde tertiaire dans l'étape (2) est de 20 % ou moins.

4. Procédé selon la revendication 1, 2 ou 3, dans lequel un sel d'ammonium quaternaire organique et une base sont utilisés dans l'étape (2).

5. Procédé selon la revendication 1, 2 ou 3, dans lequel un hydroxyde d'ammonium quaternaire organique est utilisé avec une base dans l'étape (2).

6. Procédé selon la revendication 1, 2, 3, 4 ou 5, dans lequel le catalyseur d'hydrogénation est un catalyseur au Pd ou un catalyseur cuivre-chrome dans l'étape (4).
